# EUROPEAN PATENT SPECIFICATION

(11) **EP 3 709 321 B1**
(45) Date of publication and mention of the grant of the patent: **26.06.2024**
(21) Application number: 20162671.0
(22) Date of filing: 12.03.2020
(51) Int. Cl.: H01G 9/048, A61N 1/39, H01G 4/255, H01M 4/70, H01G 9/052, A61N 1/36, A61N 1/378, H01G 11/70, H01G 11/26, H01G 11/86

(54) **ELECTRODE ELEMENT FOR A CAPACITOR, CAPACITOR AND METHOD FOR PRODUCING THE ELECTRODE ELEMENT**
ELEKTRODENELEMENT FÜR EINEN KONDENSATOR, KONDENSATOR UND VERFAHREN ZUR HERSTELLUNG DES ELEKTRODENELEMENTS
ÉLÉMENT D'ÉLECTRODE POUR UN CONDENSATEUR, CONDENSATEUR ET PROCÉDÉ DE PRODUCTION DE L'ÉLÉMENT D'ÉLECTRODE

(30) Priority: 15.03.2019 DE 102019106677
(43) Date of publication of application: 16.09.2020
(60) Divisional application: 24176235.0
(73) Proprietor: BIOTRONIK SE & Co. KG, 12359 Berlin (DE)
(72) Inventor: DÖRR, Thomas, 12437 Berlin (DE); WEISS, Ingo, 12435 Berlin (DE)
(74) Representative: Biotronik Corporate Services SE

(56) References cited:
- EP-A1- 3 193 392
- EP-A2- 0 926 932
- EP-A2- 1 111 694
- DE-A1-102011 089 174
- JP-A- H04 127 512
- JP-A- 2001 284 166
- US-A- 5 949 639
- US-A1- 2016 013 469
- US-B1- 6 416 559
- US-B1- 6 421 226

## Description

The invention relates to an electrode element for an energy storage unit, in particular for use in an implantable electrotherapeutic device, to a method for producing an electrode element, to an energy storage unit, in particular for use in an implantable electrotherapeutic device, and to an implantable electrotherapeutic device, in particular an impulse generator such as, for example, a cardiac pacemaker, an implantable cardioverter-defibrillator, or a neurostimulator.

Suitable energy storage units for implantable electrotherapeutic devices are, for example, primary and secondary batteries, for example, such as lithium batteries or lithium ion batteries and capacitors, such as e.g. electrolytic capacitors. Such energy storage units include electrodes having an electrode body made of an active electrode material.

For example, US 6,421,226 A discloses an aluminum electrolytic capacitor for an implantable defibrillator with aluminum foils having perforations and cavities.

As another example, a battery anode component is known from document DE 10 2011 089 174 A1, that at has least two spatially separated recesses as lithium receiving chambers that are separated from one another by a current collector component, wherein a predetermined breaking point is embodied in the current collector component between two adjacent lithium receiving chambers. Because of this, only very little lithium is released if the component is mechanically destroyed.

However, in production methods for energy storage units according to the prior art, the activity of the electrodes varies relatively significantly, and thus the storage capacity of the energy storage units in which the electrodes are installed, due to fluctuations in the quality of the raw materials and due to process fluctuations during production of the electrodes.

In order to ensure the required tolerance in the storage capacity within a prespecified specification, the raw materials are selected and if necessary the filling mass of the electrode bodies with active electrode material is varied. This has the disadvantage that the selection is generally made experimentally using test assemblies having many parts that, depending on the results, cannot be used further in end products and therefore production costs are significantly higher. If the filling mass is varied, depending on the electrode density to be attained, its external dimensions may vary enough that the fit during assembly of the energy storage unit is no longer accurate. Likewise, in order to attain a prespecified density with a modified filling mass, the shape and therefore also the tools (at least the tool paths) must be adjusted. The production and modification of such tools is very expensive and corrections and modifications generally require modifications going all the way back to the design process

It is therefore the objective of the present invention to provide an electrode element for an energy storage unit, a method for producing an electrode element, an energy storage unit, and an implantable electrotherapeutic device having an inventive energy storage unit that are improved with respect to the described drawbacks of the prior art. It is in particular the object of the present invention to provide an energy storage unit, in particular for an implantable electrotherapeutic device, which energy storage unit has few production-caused fluctuations in its storage capacity and which can be produced in a cost effective manner.

This objective is attained by an electrode element according to claim 1, a manufacturing method according to claim 5, an electrolytic capacitor according to claim 10, and an implantable electrotherapeutic device according to claim 11. Advantageous embodiments of the invention are provided in dependent claims and are described in the following.

A first aspect of the invention relates to an electrode element for electrolytic capacitor having a liquid electrolyte in particular for use in an implantable electrotherapeutic device, wherein the electrode element has an electrode body that is formed from an active electrode material selected from tantalum and niobium.

According to the invention, it is particularly envisioned that the electrode body comprises at least one recess on its surface or in its interior, wherein the mass of the electrode body is adjusted or is adjustable, in particular to a desired value, by the recess.

Particularly, the electrode element further comprises a terminal lead being electrically connected to the electrode body.

By the at least one recess or using the at least one partial volume of lower density, the mass of the active electrode material is reduced compared to an electrode element not having the recess or not having the partial volume of lower density. Likewise, the accessible surface of the electrode body may be reduced in a controlled manner using the cover material.

Because of this, when the electrode element is used in an electrolytic capacitor, the storage capacity of the capacitor unit may advantageously be adjusted in a simple manner and with good accuracy, since this storage capacity is a function of the mass of the electrode body or of its surface area that can be wetted by an electrolyte. This makes it possible to compensate fluctuations in the properties of the raw materials used or fluctuations in the processing parameters in further production steps. Thus, complex tests required in production methods according to the prior art and expensive new acquisition or modification of tools is not necessary. Thereby, energy storage units with low tolerances in the storage capacity can be produced with low production costs.

In the context of the present invention, an electrode element shall be construed to be a component that is suitable as an electrode, in particular an anode, in an electrolytic capacitor. The electrode comprises a cohesive electrode body in any desired shape and made of an active electrode material, but may have additional components (e.g. cover material, filler material) in addition to the electrode body.

Depending on the material properties of the active electrode material, the mass of the active electrode material, and the shape of the electrode body, the electrode element has a certain activity, i.e., a specific tendency to receive or release charge carriers. The higher this activity is, the greater the storage capacity of an energy storage unit in which the electrode element is used as electrode, in particular as an anode.

The recess of the electrode element is a depression in the electrode body, e.g. a hollow chamber in the interior of the electrode body, a blind hole on the surface of the electrode body, or a through-hole. The recess may have any shape, e.g. it may have a polygonal or round or oval-shaped cross-section or may be rounded. Of course a plurality of recesses may be provided in the electrode body, as well, wherein different shapes and sizes may also be combined with one another, e.g. blind holes and through-holes or hollow chambers in the interior and recesses on the surface. The recess or recesses are added to the electrode body in a controlled manner, in particular during the production process. Adding recesses has the additional advantage that because of this the activity of the electrode element may be adjusted in a particularly simple and variable manner.

According to one embodiment, the recess forms a separation point, the separation point being designed such that the electrode body may be mechanically separated at the separation point into a first segment and a second segment.

The mechanical separation effects the controlled reduction in the mass of the active electrode material. Following separation, the first segment and/or the second segment may continue to be used as separate electrode elements, e.g., in an inventive energy storage unit.

This has the advantage that, even after the conclusion of the essential production steps for the electrode element, its activity may be adjusted in a simple and easily controlled manner, e.g., during assembly of an energy storage unit.

The separation point may be formed in particular by the recess, e.g. a notch in the surface of the electrode body, or by a region of lower density in the active electrode material.

Of course, the separation point may be realized using one or a plurality of recesses, e.g. by arranging the recesses along a breaking edge if a plurality of recesses are used.

According to a further embodiment, the separation point is designed as a predetermined breaking point, wherein the electrode body may be mechanically separated at the target breaking point by breaking the electrode body into the first segment and the second segment.

According to a further embodiment, the electrode body comprises at least a first partial body, a second partial body, and a connecting element, wherein the first partial body, the second partial body, and the connecting element are each made of the active electrode material, the first partial body and the second partial body are mechanically connected to one another by the connecting element, and the connecting element is extended along a longitudinal axis from the first partial body towards the second partial body, and wherein the connecting element has a smaller cross-sectional area perpendicular to the longitudinal axis than the first partial body and the second partial body, so that the recess is formed between the first partial body, the second partial body, and the connecting element, and wherein the first partial body and the second partial body may be mechanically separated by means of severing the connecting element.

Severing the connecting element effects controlled reduction in the mass of the active electrode material. Thereby, following the completion of the essential production steps, it is possible to reduce the activity of the electrode element in a simple manner, e.g. during the assembly of an energy storage unit.

The first partial body and the second partial body and the connecting element comprise or essentially consists of the same active electrode material, e.g. tantalum or niobium. The electrode element is in particular suited for use as an electrode in an electrolytic capacitor.

The connecting elements may be, e.g., wires or links made of the active electrode material.

According to a further embodiment, the electrode body comprises at least one additional partial body and at least one additional connecting element, wherein two partial bodies are each mechanically connected by a connecting element, and wherein the additional connecting elements, perpendicular to their longitudinal axis, also have a smaller cross-sectional surface than the adjacent partial bodies, so that recesses are formed between the partial bodies and the connecting element, and wherein the partial bodies may be mechanically separated by means of severing the specific connecting element.

In such electrode bodies having more than two partial bodies, the partial bodies may be connected in a chain-like manner by means of connecting elements, or branches may be provided so that a two or three-dimensional arrangement of partial bodies results.

According to a further embodiment, a separation point or the aforesaid separation point of the electrode body is realized by the connecting element.

According to a further embodiment, the electrode element comprises a filler material, the filler material filling the recess or recesses of the electrode body.

The filler material may be in the interior of the electrode body and, e.g., in the shape of spheres having a diameter of 0.1 µm to 3 mm (dimensions are preferably 0.1-50 µm or 100-1000 µm) or may be particles having a mean diameter or mean grain size in the order of magnitude of the powder size of a raw material of the active electrode material. Typically, a raw material powder having a mean grain size of less than 0.6 mm is used, preferably in the range of 75 µm to 150 µm. In the context of the present invention, the term mean diameter or grain size refers in particular to the arithmetic mean of all diameters of the particles or to the median of the size distribution of all particles.

The filler material is in particular ceramic or glass.
- A second aspect of the invention relates to a method for manufacturing an electrode element for an electrolytic capacitor, in particular according to the first aspect of the invention, wherein an electrode body is made of an active electrode material, in particular in a shaping step, and wherein the mass of the active electrode material of the electrode body is reduced.

A shaping step shall be construed to mean any production step in which the electrode body receives its shape. This may be, e.g., a metal casting method, a sintering method, a punch method, or a cutting method.

According to the invention it is particularly envisioned that the mass of the electrode body is reduced, e.g., by adding a recess to the electrode body during or after the shaping step. In this way, the activity of the electrode element may advantageously be adjusted such that an energy storage unit that uses the electrode element as an electrode obtains a desired storage capacity. The activity of the electrode element may be adjusted during the production, e.g. by machining out a defined volume in order to adjust the mass of the electrode body or, e.g. using one or a plurality of recesses, a separation point may be added to the electrode body so that after the completion of the essential production step, the activity of the electrode element may be adjusted by separating at the separation point.

According to one embodiment of the method of the invention, the active electrode material is electrically connected to a terminal lead.

According to one embodiment of the method, the electrode body, in particular the metal electrode body, is pre-processed by etching.

According to further embodiment of the method, the electrode body, in particular the metal electrode body, is shaped during the shaping step by punching and/or cutting (e.g. by means of laser, water jet, electron beam, or sawing).

According to a further embodiment, the electrode body, in particular the metal electrode body, is shaped, and in particular sintered, during the shaping step by pressing powder raw material (with or without binding agent).

According to a further embodiment, a recess is formed on the surface of the electrode body or in the interior of the electrode body, wherein the mass of the active electrode material of the electrode body is reduced by formation of the recess.

According to a further embodiment, the recess is formed after the electrode body has been formed, in particular after the conclusion of the shaping step, by removing active electrode material from the electrode body.

The active electrode material may be removed, e.g. by drilling, milling, or punching. The formed recess may be, e.g. a blind hole or a through-hole.

Alternatively, the active electrode material may also be removed, e.g. by producing a notch in the outer contour of the electrode body or by separating, in particular sawing, grinding, or breaking, a part of the electrode body.

According to a further embodiment of the method, the active electrode material is a valve metal, wherein the recess is formed following formation of a metal oxide coating of the valve metal. This formation of the metal oxide coating is also called forming and occurs in particular by means of anodic oxidation of the valve metal. In particular, following formation of the recess, a metal oxide coating of the valve metal is formed again on the recess by anodic oxidation.

The term valve metal shall be construed to mean a metal that, through anodic oxidation, forms a coating of metal oxide that is electrically non-conducting. Such valve metals are in particular aluminum, tantalum, niobium, and zirconium. These are used, e.g. for anodes of electrolytic capacitors, the metal oxide coating formed functioning as dielectric material.

According to a further embodiment of the method, during the formation of the electrode body, in particular during the shaping step, the recess is formed by displacing active electrode material.

This is possible in particular by pressing-in recesses like holes (blind hole or through-hole or indentations, in particular having a rounded surface shape). The recesses are in particular designed such that the nominal outer dimensions of the electrode body are retained.

The pressing-in is accomplished in particular by means of a stamp, in particular by means of an adjustable stamp.

The electrode body is likewise formed in particular by pressing, wherein the pressing-in of the recesses is accomplished simultaneously or in one production step with the pressing of the electrode body.

According to a further embodiment of the method, a separation point of the electrode body is generated by the recess, wherein the electrode body is or may be mechanically separated at the separation point into a first segment and a second segment.

The separation point may be realized using one or a plurality of recesses.

Of course it is also possible for a plurality of separation points in the active electrode material to be realized using recesses.

According to another embodiment, the active electrode material is a valve metal, wherein the electrode body is mechanically separated into a first segment and a second segment or additional segments at the separation point following formation of a metal oxide coating of the valve metal, that is, after the so-called forming. In particular, following the aforesaid mechanical separation at the separation point, a metal oxide coating of the valve metal is again formed.

According to another embodiment, at least a first partial body, a second partial body, and a connecting element are provided, wherein the first partial body, the second partial body, and the connecting element are each made of the active electrode material, and wherein the first partial body and the second partial body are mechanically connected to one another by the connecting element, so that the electrode element is formed, and wherein the connecting element is extended along a longitudinal axis from the first partial body towards the second partial body, and wherein the connecting element has a smaller cross-sectional area perpendicular to the longitudinal axis than the first partial body and the second partial body, so that the recess is formed between the first partial body, the second partial body, and the connecting element, and wherein the first partial body and the second partial body may be or are mechanically separated by means of severing the connecting element. That is, the separation may be part of the production method or may occur after the conclusion of the manufacturing method.

Wires or links made of the active electrode material may be used as connecting elements, for example.

According to a further embodiment, at least one other partial body and at least one other connecting element are provided, wherein at least two partial bodies are mechanically connected using a connecting element, and wherein the other connecting elements also have a smaller cross-sectional area perpendicular to their longitudinal axis than the adjacent partial body, so that a recess is formed between each partial body and connecting element, and wherein the partial bodies are or may be mechanically separated by means of severing the specific connecting element. That is, the separation may be part of the production method or may occur after the conclusion of the production method.

According to a further embodiment, the active electrode material is a valve metal, wherein the connecting element is severed following formation of a metal oxide coating of the valve metal, that is, after the so-called forming. In particular, a metal oxide coating of the valve metal is formed again following the aforesaid severing at the severed point of the connecting element.

According to another embodiment, a separation point for the electrode body is realized by the connecting element.

According to a further embodiment, the recess or recesses are filled with a filler material. This in particular may stabilize the mechanical structure of the electrode body. If the electrode element is used as an electrode in a capacitor, in particular in an electrolytic capacitor, the filler material is designed in particular such that it does not negatively impact the functioning of the capacitor.

According to a further embodiment, the filler material is added to the electrode body during the formation of the electrode body, that is, during the shaping step, or after the formation of the electrode body, that is, after the completion of the shaping step. This may be accomplished, e.g., by pressing the filler material into the electrode body. The filler material may be added to the electrode body, e.g., in the shape of spheres having a diameter of 0.1 µm to 3 mm (preferred dimensions are 0.1-50 µm or 100-1000 µm) or as particles having a mean diameter in the order of magnitude of the powder size of a raw material of the active electrode material, for example less than 0.6 mm, in particular in the range of 75 µm to 150 µm. The filler material may be ceramic or glass.

According to a further embodiment, an activity of the electrode element is measured, wherein after the activity is measured the recess or the partial volume of lower density is formed such that or the cover material is applied such that the activity of the electrode element is adjusted to a target value.

This may be controlled, e.g., by remeasuring the activity after the formation of the recess. Alternatively, the control measurement may also occur only in a random sample of the produced electrode elements, that is, the calibration occurs based on random samples and is applied by lot.

The activity of the electrode element may occur, e.g., by determining a storage capacity of an electrolytic capacitor according to the invention in which the electrode element forms an electrode. Alternatively, this measurement may also be taken indirectly, e.g. by determining the mass of the active electrode material.

A third aspect of the invention relates to an electrolytic capacitor, in particular to use in an implantable electrotherapeutic device, wherein the electrolytic capacitor has at least one electrode element according to the first aspect of the invention, and wherein the electrode element forms an anode, of the electrolytic capacitor.

With this, the active electrode material comprises in particular tantalum, or niobium.

A fourth aspect of the invention relates to a method for manufacturing an electrolytic capacitor, in particular according to the third aspect of the invention, wherein the electrolytic comprises an electrode element, in particular according to the first aspect of the invention, that is produced with a method according to the second aspect of the invention, wherein the electrode element forms an anode, of the electrolytic capacitor, and wherein a storage capacity of the electrolytic capacitor is adjusted or calibrated by means of reducing the mass of the active electrode material of the electrode body.

According to one embodiment of the method for producing an energy storage unit, correction of the storage capacity of the energy storage unit occurs by less than 20%, in particular by less than 5%.

A fifth aspect of the invention relates to an implantable electrotherapeutic device, in particular an impulse generator, wherein the implantable electrotherapeutic device comprises at least one electrolytic capacitor according to the third aspect of the invention. The electrolytic capacitor acts in particular as an energy source for operating the implantable electrotherapeutic device. The impulse generator is in particular a cardiac pacemaker, an implantable cardioverter-defibrillator (ICD), or a neurostimulator.

Exemplary embodiments of the invention are described in the following, referring to figures. The figures assist in explaining the invention, but do not limit the protective scope.
- Fig. 1: is an electrode element according to the invention having recesses in the electrode body;
- Fig. 2: illustrates an exemplary embodiment of the electrode element according to the invention having a recess and provides a schematic depiction of one embodiment of the inventive method;
- Fig. 3: illustrates an embodiment of the electrode element according to the invention having a separation point and provides a schematic depiction of another embodiment of the inventive method;
- Fig. 4: illustrates another embodiment of the electrode element according to the invention, having partial bodies and connecting elements;
- Fig. 5: illustrates another embodiment of the inventive electrode element having recesses;
- Fig. 6: illustrates another embodiment of the inventive electrode element having particles made of a filler material in the interior of the electrode body;
- Fig. 7: illustrates an electrode element having a partial volume of lower density;
- Fig. 8: illustrates an electrode element having a coating made of a cover material;
- Fig. 9: illustrates an electrolytic capacitor.

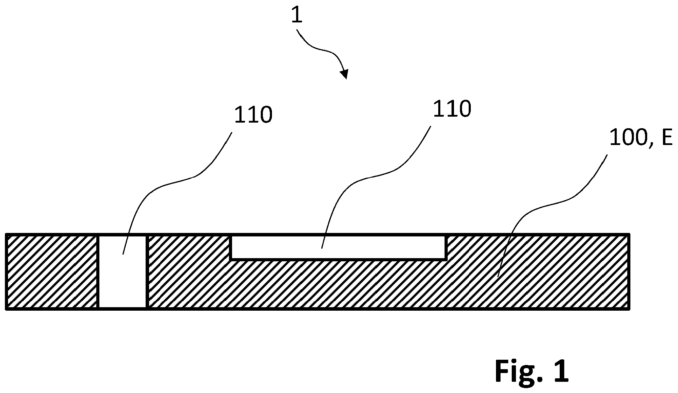

In detail, Figure 1 is a sectional illustration of an electrode element 1 having a cohesive electrode body 100. The electrode body 100 is made of an active electrode material E. In the exemplary embodiment depicted, the electrode body 100 has two recesses 110 or cavities, wherein one of the recesses 110 is designed as a blind hole, that is, a cavity that does not go all the way through, and the other recess 110 is embodied as a through-hole, that is, as a cavity that does go all the way through.

By the recess 110, the activity of the electrode element 1 may be adjusted to a desired value as early as during manufacturing of the electrode body 100 by reducing the mass of the electrode body 100. The activity of the electrode element 1 means the tendency to receive or release charge carriers, this tendency leading to a specific storage capacity of an energy storage unit such as a capacitor if the electrode element 1 is used as an electrode, in particular as an anode. This activity is in particular a function of the mass of the electrode body 100 or of the surface area of the electrode body 100 accessible for an electrolyte.

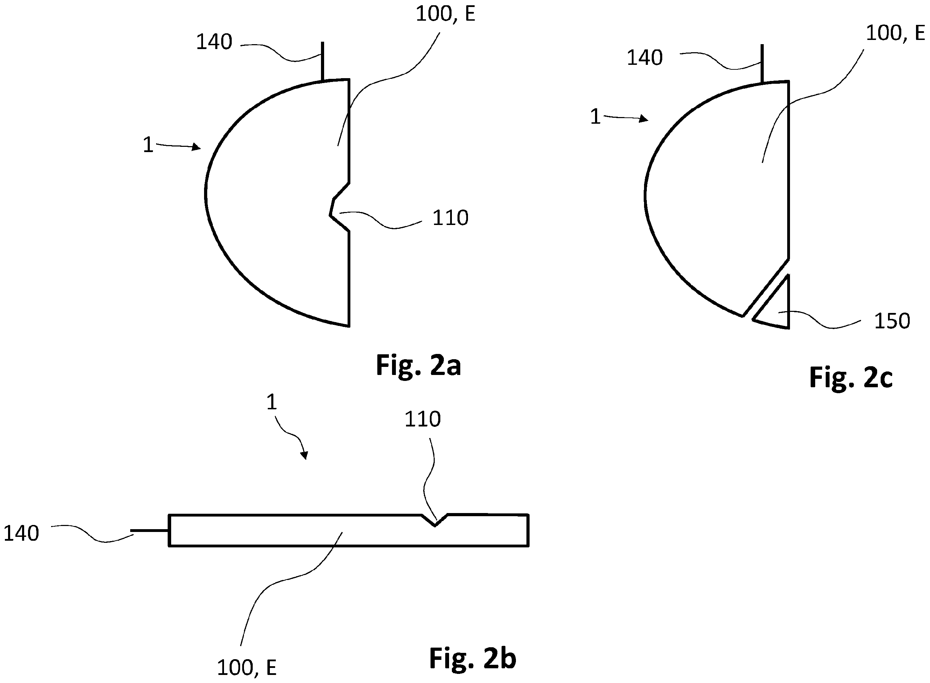

Figure 2 depicts a typical realization of the electrode element 1 of the invention. The latter comprises an electrode body 100 and a connecting pin 140 connected to the electrode body 100 in a conductive manner for electrically connecting the electrode element 1.

Figure 2a depicts a top view of such an electrode element 1. The electrode body 100 made of the active electrode material E has an essentially semi-circular shape, wherein a recess 110 in the form of a semi-circle has been introduced to the straight side of the semi-circle. Figure 2b is a sectional view of an electrode element 1 shaped as in Figure 2a.

Figure 2c provides a schematic depiction of the result of one manufacturing method of the invention for the electrode element 1. In this method, first the electrode body 100 is made from the active electrode material E and connected to the connecting pin 140. The electrode body 100 again is semi-circular in shape. Following this manufacturing step, an electrode piece 150 is separated from the electrode body 100, for example by cutting, sawing, or milling. In this way, the mass of the active electrode material E is reduced in a controlled manner in order to obtain an appropriate desired electrode activity when the electrode element 1 is used in an energy storage unit. Thus, no recess 110 is introduced to the electrode body 100 in this embodiment of the method.

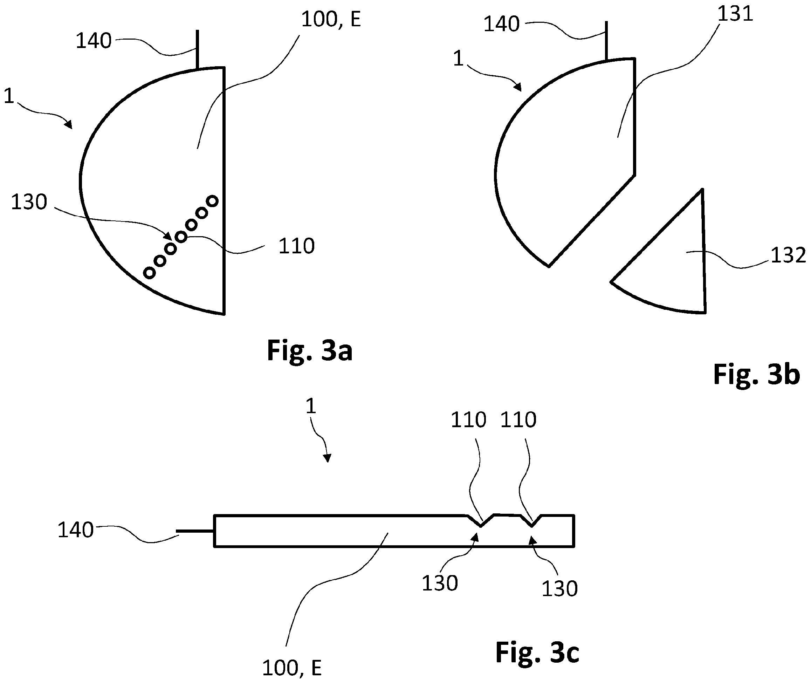

Figure 3 depicts a further embodiment of the electrode element 1 of the invention in a top view (Figures 3a and 3b) and in section (Figure 3c).

Figure 3a depicts the electrode element 1 prior to a separating process and Figure 3b depicts the electrode element 1 following the separating process. In the top view, the electrode element 1 is essentially semi-circular in shape and comprises a cohesive electrode body 100 made of an active electrode material E and a connecting pin 140. The electrode body 100 furthermore has a plurality of recesses 110 in the form of bores that are arranged along one line. The recesses 110 may be designed as through-holes or as blind holes. Together the recesses 110 form a separation point 130 or predetermined breaking point that makes it possible to mechanically separate the electrode body 100 at the separation point 130 into a first segment 131 and a second segment 132, for example by breaking. Figure 3b depicts the first segment 131 and the second segment 132 following separation at the separation point 130.

Figure 3c depicts an electrode element 1 in section, the separation points 130 or predetermined breaking points being realized using recesses 110 in the form of notches.

Due to the described separation point 130, even after the completion of the essential production steps for the electrode element 1, the mass of the electrode body 100 may be reduced by separating at the separation point 130 in order to adjust the activity of the electrode element 1 to a desired value, e.g. in order to calibrate the storage capacity of an energy storage unit in which the electrode element 1 is used as an electrode, in particular an anode. One of the segments 131, 132 may then further be used as an electrode element 1 having reduced mass and activity, in this case preferably the first segment 131, which in this case already has a connecting pin 140.

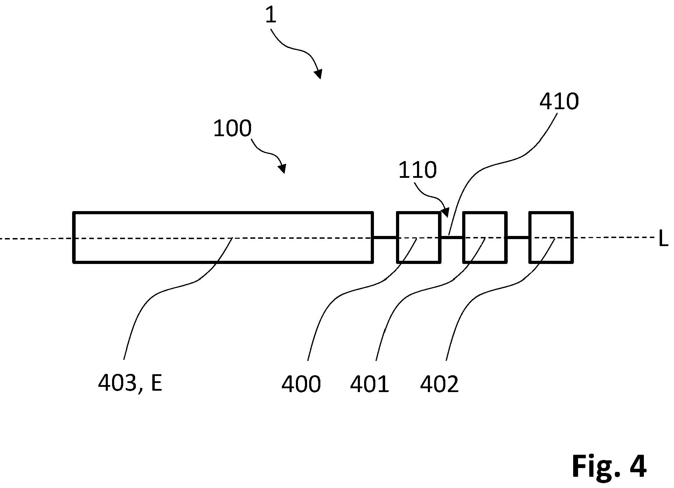

Figure 4 depicts a sectional view of an exemplary embodiment of the electrode element 1 of the invention, which in this case has already been produced with segments 400, 401, 402, 403 made of the active electrode material E, the segments 400, 401, 402, 403 being connected to one another via connecting elements 410. The connecting elements 410 are made of the same active electrode material E, for example a valve metal, as the segments 400, 401, 402, 403. In the example shown here, the segments 400, 401, 402, 403 are connected to one another as a linear chain via the connecting elements 410, the connecting elements 410 extending along a common longitudinal axis L. However, it is also possible to create branched structures or a two or three-dimensional arrangement of segments of the electrode body 100.

The segments 400, 401, 402, 403 may be mechanically separated from one another at the connecting elements 410 in that the connecting elements 410 are severed. This may be accomplished, e.g. by breaking or cutting. In this way, the mass of the electrode body 100 may be advantageously reduced in a controlled and simple manner even after the conclusion of the essential production steps for the electrode element, so that the electrode activity of the electrode element 1 is adjustable, e.g., with respect to use in an energy storage unit, wherein the electrode activity influences the storage capacity of the energy storage unit there.

The connecting elements 410 may be realized as wires or links, for example.

Moreover, the connecting elements 410 have a smaller cross-sectional area perpendicular to the longitudinal axis L than the first segment 400, the second segment 401, the third segment 402, and the fourth segment 403, so that a recess 110 is created between segments 400, 401, 402, 403 and each connecting element 410.

It should be noted that the connecting elements 410 in this embodiment realize separation points in the context of this invention.

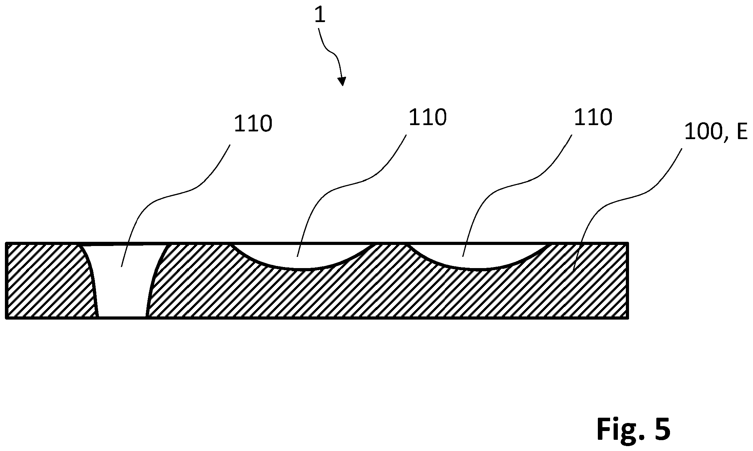

Figure 5 depicts a section through an electrode body 100 of an inventive electrode element 1 having in particular pressed-in recesses 110 in the form of cavities. One of these recesses 110 is designed such that it passes through, while the other two recesses 110 are designed as depressions in the surface of the electrode body 100. Such recesses 110 may be introduced in particular during a shaping step in the production of the electrode body 100, e.g., by pressing into the electrode body 100 by means of a stamp.

By selecting the dimensioning of the recesses, the mass of the active electrode material E may then be adjusted to a desired value in order to attain a desired activity despite fluctuations in the raw materials and previous production steps. This activity then leads in particular to a desired storage capacity of an energy storage unit in which the electrode element 1 is used.

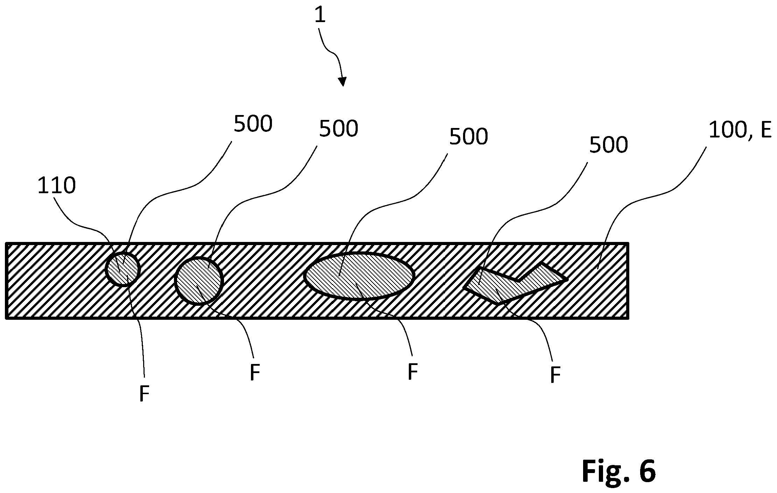

Figure 6 is a sectional depiction of an electrode element 1 of the invention that has recesses 110 in the interior of the electrode bod 100. These recesses 110 are filled with particles 500 of a filler material F. This filler material F is not the same material as the active electrode material E and in particular does not have any active electrode properties. The particles 500 may be pressed into the electrode body 100, for example, during a shaping step for the latter. The particles 500 may have different shapes and different sizes. In particular, the particles 500 may also occupy different positions in different electrode bodies 100.

Of course the filler material F does not have to be in the form of particles 500, but instead may be poured in liquid form into the recesses 110 provided on the surface of the electrode body 100.

Due to the filler material F, the mass of the active electrode material E and thus the activity of the electrode element 1 may advantageously be adjusted to a desired value, since active electrode material E is displaced by the filler material F. At the same time, the mechanical stability of the electrode body 100 is increased due to the filler material F in the recesses 110.

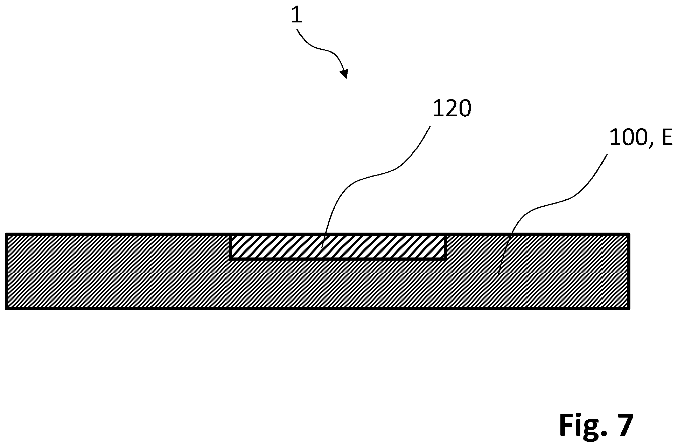

Figure 7 is a sectional depiction of another electrode element 1 in which the electrode body 100 has on its surface a partial volume 120 of lower density. The electrode body 100 comprises the same active electrode material E both within and outside of the partial volume 120, wherein however the density of the active electrode material E within the partial volume 120 is lower than outside the partial volume 120.

Thereby, the activity of the electrode element 1 is reduced compared to an electrode element 1 having uniformly higher density, so that when the electrode element 1 is used as an electrode, especially an anode, in an energy storage element, it is possible to adjust a desired storage capacity by selecting the size and density of the partial volume 120.

Of course the partial volume 120 does not have to be disposed on the surface of the electrode body 100, but instead may also be arranged in the interior of the electrode body 100.

Likewise, of course, a plurality of partial volumes 120 of lower density may be provided in the electrode body 100.

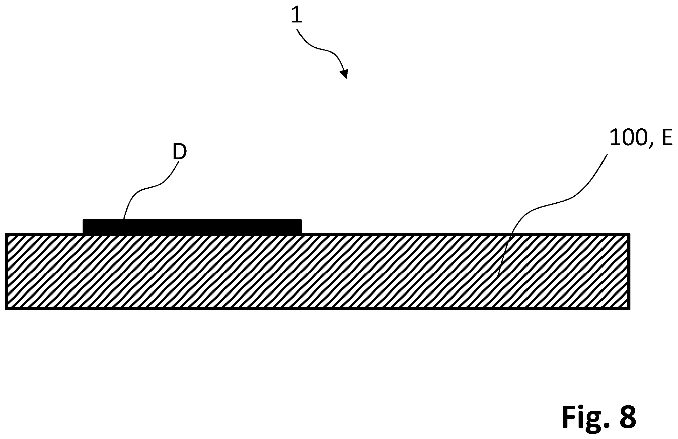

Figure 8 depicts still another electrode element 1 having an electrode body 100 made of the active electrode material E and having a cover layer made of a cover material D, for example an impregnating agent, on the surface of the electrode body 100. The cover material D causes the surface of the electrode body 100 covered by the cover material D not to be wetted with an electrolyte when the electrode body 100 is in contact with the electrolyte. Because of this, the surface covered by the cover layer cannot fulfill its function as electrode, in particular in an energy storage unit, so that the activity of the electrode element 1 is reduced, depending on the dosing and arrangement of the cover material D on the electrode body 100. Due to this, the storage capacity of an energy storage unit that uses the inventive electrode element 1 may be adjusted to a desired value.

Alternative to electrode element in Figure 8, the cover material D may also cover the surface of capillaries that run from the surface into the interior of the electrode body 100.

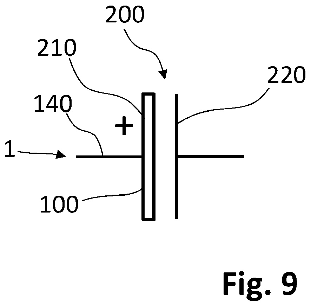

Figure 9 schematically depicts an electrolytic capacitor 200. It has a cathode 220 and, as anode 210, the electrode element 1 of the invention, wherein the electrode element 1 in this case is made of a valve metal, such tantalum, or niobium. An oxide layer of this valve metal forms the dielectric material in the case of an electrolytic capacitor.

### Reference numbers

| | |
|---|---|
| Electrode element | 1 |
| Electrode body | 100 |
| Recess | 110 |
| Partial volume | 120 |
| Separation point | 130 |
| First segment | 131 |
| Second segment | 132 |
| Connecting pin | 140 |
| Electrode part | 150 |
| Energy storage unit | 200 |
| Anode | 210 |
| Cathode | 220 |
| First partial body | 400 |
| Second partial body | 401 |
| Third partial body | 402 |
| Fourth partial body | 403 |
| Connecting element | 410 |
| Particles | 500 |
| Cover material | D |
| Active electrode material | E |
| Filler material | F |
| Longitudinal axis | L |

## Claims

1. An electrode element (1) for an electrolytic capacitor (200) having a liquid electrolyte, comprising an electrode body (100) made of an active electrode material (E) selected from tantalum and niobium and a terminal lead being electrically connected to the electrode body
**characterized in that** the electrode body (100) comprises at least one recess (110) on its surface or in its interior, wherein the mass of the electrode body (100) is adjusted or is adjustable by the recess (110).

2. The electrode element (1) according to claim 1, **characterized in that** the recess (110) forms a separation point (130), the separation point (130) being designed such that the electrode body (100) is mechanically separatable at the separation point (130) into a first segment (131) and a second segment (132).

3. The electrode element (1) according to claim 1 or 2, **characterized in that** the electrode body (100) comprises at least a first partial body (400), a second partial body (401), and a connecting element (410), wherein the first partial body (400), the second partial body (401), and the connecting element (410) are each made of the active electrode material (E), and wherein the first partial body (400) and the second partial body (401) are mechanically connected to one another by the connecting element (410), and the connecting element (410) is extended along a longitudinal axis (L) from the first partial body (400) towards the second partial body (401), and wherein the connecting element (410) has a smaller cross-sectional area perpendicular to the longitudinal axis (L) than the first partial body (400) and the second partial body (401), so that the recess (110) is formed between the first partial body (400), the second partial body (401), and the connecting element (410), and wherein the first partial body (400) and the second partial body (401) may be mechanically separated by means of severing the connecting element (410).

4. The electrode element (1) according to any of the preceding claims, **characterized in that** the electrode element (1) comprises a filler material (F), the filler material (F) filling the recess (110) of the electrode body (100).

5. A method for manufacturing an electrode element (1) for an electrolytic capacitor (200) having a liquid electrolyte according to any of claims 1 to 4, wherein an electrode body (100) is made of an active electrode material (E), and the mass of the active electrode material (E) of the electrode body (100) is reduced by forming a recess (110) on the surface of the electrode body (100) or in the interior of the electrode body (100), and wherein the mass of the active electrode material (E) of the electrode body (100) is reduced by the formation of the recess (110).

6. The method according to claim 5, wherein the recess (110) is formed after the electrode body (100) has been formed by removing active electrode material (E) from the electrode body (100).

7. The method according to claim 5, wherein the recess (110) is formed during the formation of the electrode body (100) by displacing active electrode material (E).

8. The method according to any of claims 5 to 7, wherein a separation point (130) of the electrode body (100) is formed by the recess (110), wherein the electrode body (100) is mechanically separated at the separation point (130) into a first segment (131) and a second segment (132).

9. The method according to any of claims 5 to 8, wherein at least a first partial body (400), a second partial body (401), and a connecting element (410) are provided, and wherein the first partial body (400), the second partial body (401), and the connecting element (410) are each made of the active electrode material (E), and wherein the first partial body (400) and the second partial body (401) are mechanically connected to one another by the connecting element (410) so that the electrode element (1) is formed, wherein the connecting element (410) is extended along a longitudinal axis (L) from the first partial body (400) towards the second partial body (401), and wherein the connecting element (410) has a smaller sectional surface area perpendicular to the longitudinal axis (L) than the first partial body (400) and the second partial body (401), so that the recess (110) is formed between the first partial body (400), the second partial body (401), and the connecting element (410), and wherein the first partial body (400) and the second partial body (401) are mechanically separated by means of severing the connecting element (410).

10. An electrolytic capacitor (200), wherein the capacitor (200) has at least one electrode element (1) according to any of claims 1 to 4 and a liquid electrolyte, and wherein the electrode element (1) forms an anode (210) of the capacitor (200).

11. An implantable electrotherapeutic device, in particular an impulse generator, wherein the implantable electrotherapeutic device has at least one capacitor (200) according to claim 10.

## Patentansprüche

1. Elektrodenelement (1) für einen Elektrolytkondensator (200) mit Flüssigelektrolyt, einen Elektrodenkörper (100) aus einem aktiven Elektrodenmaterial (E), ausgewählt aus Tantal und Niob, und einen Anschlussleiter, die mit dem Elektrodenkörper verbunden ist, **dadurch gekennzeichnet, dass** der Elektrodenkörper (100) mindestens eine Aussparung (110) an seiner Oberfläche oder in seinem Inneren umfasst, wobei die Masse des Elektrodenkörpers (100) durch die Aussparung (110) angepasst ist oder anpassbar ist.

2. Elektrodenelement (1) nach Anspruch 1, **dadurch gekennzeichnet, dass** die Aussparung (110) eine Trennstelle (130) bildet, wobei die Trennstelle (130) so ausgebildet ist, dass der Elektrodenkörper (100) an der Trennstelle (130) mechanisch in ein erstes Segment (131) und ein zweites Segment (132) teilbar ist.

3. Elektrodenelement (1) nach Anspruch 1 oder 2, **dadurch gekennzeichnet, dass** der Elektrodenkörper (100) mindestens einen ersten Teilkörper (400), einen zweiten Teilkörper (401) und ein Verbindungselement (410) umfasst, wobei der erste Teilkörper (400), der zweite Teilkörper (401) und das Verbindungselement (410) jeweils aus dem aktiven Elektrodenmaterial (E) bestehen, und wobei der erste Teilkörper (400) und der zweite Teilkörper (401) mechanisch miteinander über das Verbindungselement (410) verbunden sind, und das Verbindungselement (410) entlang einer Längsachse (L) vom ersten Teilkörper (400) hin zum zweiten Teilkörper (401) verläuft, und wobei das Verbindungselement (410) einen kleineren Querschnittsbereich senkrecht zur Längsachse (L) als der erste Teilkörper (400) und der zweite Teilkörper (401) hat, sodass die Aussparung (110) zwischen dem ersten Teilkörper (400), dem zweiten Teilkörper (401) und dem Verbindungselement (410) ausgebildet ist, und wobei der erste Teilkörper (400) und der zweite Teilkörper (401) durch Abtrennen des Verbindungselements (410) mechanisch geteilt werden können.

4. Elektrodenelement (1) nach einem der vorstehenden Ansprüche, **dadurch gekennzeichnet, dass** das Elektrodenelement (1) einen Füllstoff (F) umfasst, wobei der Füllstoff (F) die Aussparung (110) des Elektrodenkörpers (100) füllt.

5. Verfahren zur Herstellung eines Elektrodenelements (1) für einen Elektrolytkondensator (200) mit Flüssigelektrolyt nach einem der Ansprüche 1 bis 4, wobei ein Elektrodenkörper (100) aus einem aktiven Elektrodenmaterial (E) hergestellt wird, und die Masse des aktiven Elektrodenmaterials (E) des Elektrodenkörpers (100) reduziert wird, indem eine Aussparung (110) an der Oberfläche des Elektrodenkörpers (100) oder im Inneren des Elektrodenkörpers (100) ausgebildet wird, und wobei die Masse des aktiven Elektrodenmaterials (E) des Elektrodenkörpers (100) durch das Ausbilden der Aussparung (110) reduziert wird.

6. Verfahren nach Anspruch 5, wobei die Aussparung (110) ausgebildet wird, nachdem der Elektrodenkörper (100) ausgebildet worden ist, indem aktives Elektrodenmaterial (E) aus dem Elektrodenkörper (100) entfernt wird.

7. Verfahren nach Anspruch 5, wobei die Aussparung (110) während der Ausbildung des Elektrodenkörpers (100) ausgebildet wird, indem aktives Elektrodenmaterial (E) verschoben wird.

8. Verfahren nach einem der Ansprüche 5 bis 7, wobei eine Trennstelle (130) des Elektrodenkörpers (100) durch die Aussparung (110) ausgebildet wird, wobei der Elektrodenkörper (100) an der Trennstelle (130) mechanisch in ein erstes Segment (131) und ein zweites Segment (132) geteilt ist.

9. Verfahren nach einem der Ansprüche 5 bis 8, wobei zumindest ein erster Teilkörper (400), ein zweiter Teilkörper (401) und ein Verbindungselement (410) bereitgestellt werden, und wobei der erste Teilkörper (400), der zweite Teilkörper (401) und das Verbindungselement (410) jeweils aus dem aktiven Elektrodenmaterial (E) bestehen, und wobei der erste Teilkörper (400) und der zweite Teilkörper (401) über das Verbindungselement (410) mechanisch miteinander verbunden sind, sodass das Elektrodenelement (1) gebildet wird, wobei das Verbindungselement (410) entlang einer Längsachse (L) vom ersten Teilkörper (400) hin zum zweiten Teilkörper (401) verläuft und wobei das Verbindungselement (410) einen kleineren Querschnittsbereich senkrecht zur Längsachse (L) hat als der erste Teilkörper (400) und der zweite Teilkörper (401) hat, sodass die Aussparung (110) zwischen dem ersten Teilkörper (400), dem zweiten Teilkörper (401) und dem Verbindungselement (410) ausgebildet ist, und wobei der erste Teilkörper (400) und der zweite Teilkörper (401) durch Abtrennen des Verbindungselements (410) mechanisch geteilt werden.

10. Elektrolytkondensator (200), wobei der Kondensator (200) mindestens ein Elektrodenelement (1) nach einem der Ansprüche 1 bis 4 und ein Flüssigelektrolyt aufweist und wobei das Elektrodenelement (1) eine Anode (210) des Kondensators (200) bildet.

11. Implantierbare elektrotherapeutische Vorrichtung, insbesondere ein Impulsgenerator, wobei die implantierbare elektrotherapeutische Vorrichtung mindestens einen Kondensator (200) nach Anspruch 10 aufweist.

## Revendications

1. Élément d'électrode (1) pour un condensateur électrolytique (200) ayant un électrolyte liquide, comprenant un corps d'électrode (100) fabriqué en un matériau d'électrode actif (E) choisi parmi le tantale et le niobium et un conducteur de borne électriquement relié au corps d'électrode
**caractérisé en ce que** le corps d'électrode (100) comprend au moins un évidement (110) sur sa surface ou dans son intérieur, dans lequel la masse du corps d'électrode (100) est ajustée ou est ajustable par l'évidement (110).

2. Élément d'électrode (1) selon la revendication 1, **caractérisé en ce que** l'évidement (110) forme un point de séparation (130), le point de séparation (130) étant conçu de telle sorte que le corps d'électrode (100) peut être séparé mécaniquement au niveau du point de séparation (130) en un premier segment (131) et un second segment (132).

3. Élément d'électrode (1) selon la revendication 1 ou 2, **caractérisé en ce que** le corps d'électrode (100) comprend au moins un premier corps partiel (400), un second corps partiel (401) et un élément de liaison (410), dans lequel le premier corps partiel (400), le second corps partiel (401) et l'élément de liaison (410) sont chacun fabriqués en le matériau d'électrode actif (E), et dans lequel le premier corps partiel (400) et le second corps partiel (401) sont mécaniquement reliés l'un à l'autre par l'élément de liaison (410), et l'élément de liaison (400) est étendu le long d'un axe longitudinal (L) à partir du premier corps partiel (400) vers le second corps partiel (401), et dans lequel l'élément de liaison (410) a une aire de section transversale perpendiculaire à l'axe longitudinal (L) plus petite que le premier corps partiel (400) et le second corps partiel (401), de telle sorte que l'évidement (110) est formé entre le premier corps partiel (400), le second corps partiel (401) et l'élément de liaison (410), et dans lequel le premier corps partiel (400) et le second corps partiel (401) peuvent être mécaniquement séparés en coupant l'élément de liaison (410).

4. Élément d'électrode (1) selon l'une quelconque des revendications précédentes, **caractérisé en ce que** l'élément d'électrode (1) comprend un matériau de remplissage (F), le matériau de remplissage (F) remplissant l'évidement (110) du corps d'électrode (100).

5. Procédé de fabrication d'un élément d'électrode (1) pour un condensateur électrolytique (200) ayant un électrolyte liquide selon l'une quelconque des revendications 1 à 4, dans lequel un corps d'électrode (100) est fabriqué en un matériau d'électrode actif (E), et la masse du matériau d'électrode actif (E) du corps d'électrode (100) est réduite en formant un évidement (110) à la surface du corps d'électrode (100) ou dans l'intérieur du corps d'électrode (100), et dans lequel la masse du matériau d'électrode actif (E) du corps d'électrode (100) est réduite par la formation de l'évidement (110).

6. Procédé selon la revendication 5, dans lequel l'évidement (110) est formé après que le corps d'électrode (100) a été formé en retirant du matériau d'électrode actif (E) du corps d'électrode (100).

7. Procédé selon la revendication 5, dans lequel l'évidement (110) est formé pendant la formation du corps d'électrode (100) en déplaçant le matériau d'électrode actif (E).

8. Procédé selon l'une quelconque des revendications 5 à 7, dans lequel un point de séparation (130) du corps d'électrode (100) est formé par l'évidement (110), dans lequel le corps d'électrode (100) est mécaniquement séparé au niveau du point de séparation (130) en un premier segment (131) et un second segment (132).

9. Procédé selon l'une quelconque des revendications 5 à 8, dans lequel au moins un premier corps partiel (400), un second corps partiel (401) et un élément de liaison (410) sont fournis, et dans lequel le premier corps partiel (400), le second corps partiel (401) et l'élément de liaison (410) sont chacun fabriqués en le matériau d'électrode actif (E), et dans lequel le premier corps partiel (400) et le second corps partiel (401) sont mécaniquement reliés l'un à l'autre par l'élément de liaison (410) de telle sorte que l'élément d'électrode (1) est formé, dans lequel l'élément de liaison (410) est étendu le long d'un axe longitudinal (L) à partir du premier corps partiel (400) vers le second corps partiel (401), et dans lequel l'élément de liaison (410) a une aire de surface de section perpendiculaire à l'axe longitudinal (L) plus petite que le premier corps partiel (400) et le second corps partiel (401), de telle sorte que l'évidement (110) est formé entre le premier corps partiel (400), le second corps partiel (401) et l'élément de liaison (410), et dans lequel le premier corps partiel (400) et le second corps partiel (401) sont mécaniquement séparés en coupant l'élément de liaison (410).

10. Condensateur électrolytique (200), dans lequel le condensateur (200) a au moins un élément d'électrode (1) selon l'une quelconque des revendications 1 à 4 et un électrolyte liquide, et dans lequel l'élément d'électrode (1) forme une anode (210) du condensateur (200).

11. Dispositif électrothérapeutique implantable, en particulier un générateur d'impulsions, dans lequel le dispositif électrothérapeutique implantable a au moins un condensateur (200) selon la revendication 10.
